(19) Europäisches Patentamt
European Patent Office
Office européen des brevets

(11) Veröffentlichungsnummer : **0 046 497**
**B1**

(12) # EUROPÄISCHE PATENTSCHRIFT

(45) Veröffentlichungstag der Patentschrift : **28.11.84**

(51) Int. Cl.³ : **C 07 D285/06**, A 01 N 43/82

(21) Anmeldenummer : **81105382.6**

(22) Anmeldetag : **10.07.81**

(54) **N-Disubstituierte Anilinderivate, ihre Herstellung, ihre Verwendung als Mikrobizide und Mittel dafür.**

(30) Priorität : **14.08.80 DE 3030736**

(43) Veröffentlichungstag der Anmeldung : **03.03.82 Patentblatt 82/09**

(45) Bekanntmachung des Hinweises auf die Patenterteilung : **28.11.84 Patentblatt 84/48**

(84) Benannte Vertragsstaaten : **AT BE CH DE FR GB IT LI LU NL SE**

(56) Entgegenhaltungen :
**EP-A- 0 010 163**
**EP-A- 0 019 742**
**DE-A- 2 728 523**
**DE-A- 2 909 991**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Hamprecht, Gerhard, Dr.**
**Rote-Turm-Strasse 28**
**D-6940 Weinheim (DE)**
Erfinder : **Ammermann, Eberhard, Dr.**
**Sachsenstrasse 3**
**D-6700 Ludwigshafen (DE)**
Erfinder : **Pommer, Ernst-Heinrich, Dr.**
**Berliner Platz 7**
**D-6703 Limburgerhof (DE)**
Erfinder : **Plath, Peter, Dr.**
**Berner Weg 24**
**D-6700 Ludwigshafen (DE)**

**EP 0 046 497 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue N-disubstituierte Anilinderivate, Verfahren zu ihrer Herstellung, Mikrobizide, die diese Verbindungen als Wirkstoffe enthalten, sowie Verfahren zur Bekämpfung von Pilzen.

Heterocyclische Carbonsäureanilide, die mikrobizid wirksam sind, sind aus der DE-OS 25 13 732 und der DE-OS 25 13 788 bekannt. Heterocyclische Reste in diesen Verbindungen sind Pyridyl, Pyrimidinyl, Dihydropyranyl, Dihydro-1,4-oxathiinyl, Thienyl und Furyl. Die Wirkung dieser Verbindungen gegen Phytophthora ist unzureichend.

Weiter sind in der DE-OS 27 28 523 1,2,3-Thiadiazol-5-carbonsäurederivate beschrieben, welche herbizide und wachstumsregulierende Wirkungen besitzen.

Es wurde nun gefunden, daß N-disubstituierte Anilinderivate der Formel I

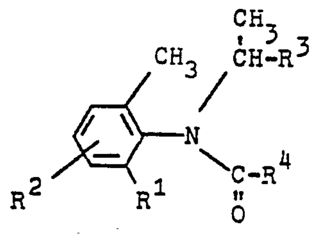

(I)

in der

$R^1$ $C_1$-$C_2$-Alkyl oder Chlor

$R^2$ Wasserstoff oder Methyl

$R^3$ $CH[OCH_3]_2$, oder $COOCH_3$ und

$R^4$ einen Thiadiazolyl-(4)- oder -(5)-rest bedeuten, mikrobizid ausgezeichnet wirksam sind und bekannten heterocyclischen Carbonsäureaniliden in ihrer Wirkung, insbesondere gegen Phytophthora, überlegen sind.

Die Herstellung der Verbindungen der Formel I erfolgt dadurch, daß man eine Verbindung der Formel II

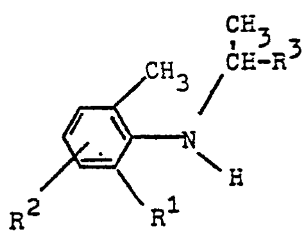

(II)

in der R, $R^1$, $R^2$ und $R^3$ die oben angegebene Bedeutung besitzen, mit einem Carbonsäurederivat der Formel III

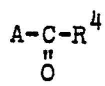

(III)

in der $R^4$ die oben angegebene Bedeutung hat und A für eine nucleophil verdrängbare Abgangsgruppe steht, umsetzt.

In der Formel III bedeutet A beispielsweise Halogen, wie Chlor oder Brom, einen Alkoxycarbonyloxyrest, wie Methoxycarbonyloxy und Ethoxycarbonyloxy, den Benzyloxycarbonyloxyrest oder einen Azolylrest, wie den Imidazolyl- oder den Triazolylrest.

Obwohl die Reaktion auch in Abwesenheit von Lösungsmitteln durchgeführt werden kann, ist es zweckmäßig, die Umsetzung in einem inerten Lösungs- oder Verdünnungsmittel vorzunehmen. Als Lösungsmittel kommen z. B. in Frage :

Halogenkohlenwasserstoffe, insbesondere Chlorkohlenwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Iodbenzol, o-, m-, p-Dichlorbenzol, o-, p-, m-Dibrombenzol, o-, m-, p-Chlortoluol, 1,2,4-Trichlorbenzol ; Ether, z. B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether ; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z. B. Ethylacetat, Acetessigester, Isobutylacetat; Amide, z. B. Formamid, Methylformamid, Dimethylformamid; Ketone, z. B. Aceton, Methylethylketon, gegebenenfalls auch Wasser und entsprechende Gemische. Zweckmäßig verwendet man das Lösungsmittel in einer Menge von 100 bis 2 000 Gew.%, vorzugsweise von 200 bis 700 Gew.%, bezogen auf Ausgangsstoff II.

Es empfiehlt sich — obwohl es nicht notwendig ist —, die Umsetzung in Gegenwart eines Säureakzeptors durchzuführen. Als solche können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. es können aber auch Zinkverbindungen verwendet werden. Als basische Verbindungen kommen z. B. in Frage: Kaliumhydroxid, Natriumhydroxid, Kaliumcarbonat, Natriumcarbonat, Lithiumhydroxid, Lithiumcarbonat, Natriumhydrogencarbonat, Kaliumhydrogencarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-secbutylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidin, N-Ethylpyrrolidon, N-Methylpiperidin, N-Ethylpiperidin, N-Methyl-pyrrolidin, N-Ethylpyrrolidin, N-methylimidazol, N-ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, -Picolin, β-Picolin, γ-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Man kann jedoch auch den bei der Reaktion entstehenden Halogenwasserstoff durch Einleiten eines Inertgases, beispielsweise Stickstoff, entfernen.

Die Reaktion kann gegebenenfalls in Gegenwart eines Reaktionsbeschleunigers vorgenommen werden. Als solche kommen vorzugsweise Metallhalogenide wie Natriumbromid oder Kaliumiodid, Azole, wie Imidazol oder 1,2,4-Triazol, Pyridine, wie 4-Dimethylaminopyridin, oder Amide, wie Dimethylformamid oder Mischungen dieser Substanzen in Betracht. Zweckmäßigerweise setzt man auf 1 Mol Anilinderivat der Formel II 0,9 bis 1,3 Mol Säurederivat der Formel III sowie gegebenenfalls 0,5 bis 1,5 Mol Base und gegebenenfalls 0,01 bis 0,1 Mol Reaktionsbeschleuniger ein.

Zweckmäßig wird das Verfahren zur Herstellung der neuen Verbindungen so durchgeführt, daß man den Ausgangsstoff II, gegebenenfalls in einem der vorgenannten Verdünnungsmittel vorlegt und dann den Ausgangsstoff III und einen Säureakzeptor gleichzeitig oder nacheinander zugibt. Man kann jedoch auch den Ausgangsstoff III in einem der vorgenannten Verdünnungsmittel vorlegen und dann den Ausgangsstoff II und einen Säureakzeptor, gleichzeitig oder in beliebiger Reihenfolge, über zwei getrennte Zuführungen zugeben.

Die Umsetzung ist in vielen Fällen nach der Zugabe der Komponenten bereits abgeschlossen, andernfalls rührt man zu ihrer Beendigung noch 10 Minuten bis 10 Stunden bei − 10 bis 120 °C, vorzugsweise 0 bis 100 °C, insbesondere 20 bis 80 °C nach.

Aus dem Reaktionsgemisch wird die Verbindung I in üblicher Weise, z. B. durch Abdestillieren von Lösungsmittel oder überschüssigem Ausgangsstoff II oder III direkt durch Absaugen isoliert. Der verbleibende Rückstand wird in diesem Fall zur Entfernung saurer oder basischer Verunreinigungen mit Wasser bzw. verdünntem Alkali oder Säure gewaschen und getrocknet. Im Fall von mit Wasser nicht mischbaren Verdünnungsmitteln kann man auch direkt das Reaktionsgemisch mit Wasser bzw. mit verdünntem Alkali oder Säure extrahieren und dann trocknen und einengen. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Chromatographie oder Destillation weiter gereinigt werden.

Die Verbindungen II lassen sich aus den entsprechenden Anilinderivaten durch Umsetzung mit Verbindungen der Formel IV

$$Hal—CH(CH_3)—R^3 \qquad (IV)$$

erhalten, worin Hal ein Halogenatom, z. B. Chlor oder Brom, bedeutet. Einzelheiten zur Herstellung der Ausgangsprodukte der Formel II kann man den Methoden entnehmen, wie sie allgemein für die Herstellung von Anilino-alkansäureestern in folgenden Publikationsorganen angegeben werden: J. Org. Chem. *30,* 4101 (1965), Tetrahedron *1967*, 487, Tetrahedron *1967*, 493.

Auch die als Ausgangsstoff eingesetzten Carbonsäurederivate der Formel III sind bekannt oder können nach bekannten Methoden hergestellt werden: J. Amer. Chem. Soc. *77*, 5359 (1955), J. Chem. Soc. *1965*, 5166.

Die Verbindungen I besitzen in der Propionat-Seitenkette ein asymmetrisches Kohlenstoffatom. Sie können auf übliche Art in die optischen Antipoden gespalten werden. Hierbei besitzt die enantiomere D-Form die stärkere mikrobizide Wirkung.

Im Rahmen der Erfindung sind demgemäß diejenigen Verbindungen, ihre Mittel und ihre Verwendung bevorzugt, welche sich auf die D-Konfiguration der Formel I beziehen.

Zur Herstellung der reinen optischen D-Antipoden wird z. B. die aus der Reaktion des Anilins mit α-Halogenpropionsäure erhaltene racemische Verbindung der Formel V

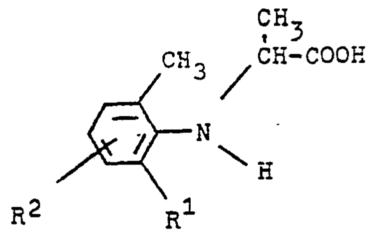

(V)

worin $R^1$ und $R^2$ die oben genannte Bedeutung haben, hergestellt und dann in an sich bekannter Weise mit einer N-haltigen optisch aktiven Base zum entsprechenden Salz umgesetzt. Durch fraktionierte Kristallisation des Salzes und nachfolgende Freisetzung der mit dem optischen D-Antipoden angereicherten Säure V und gegebenenfalls Wiederholung (auch mehrfache Wiederholung) der Salzbildung, Kristallisation und Freisetzung der α-Anilinopropionsäure V gewinnt man stufenweise die reine D-Form. Aus dieser läßt sich dann auf übliche Art, z. B. mit Methanol oder vorzugsweise mit einem Methanolat, insbesondere dem Na- oder K-Methanolat, und dem Säurehalogenid des optischen Antipoden der Formel V der optisch aktive Ester II herstellen. Dieser wird dann erfindungsgemäß mit III umgesetzt.

Als optisch aktive organische Base kommt z. B. α-Phenylethylamin in Frage.

Unabhängig von der optischen Isomerie wird in der regel eine Atropisomerie um die phenyl-N< Achse in den Fällen beobachtet, wo der Phenylring unsymmetrisch zu dieser Achse (gegebenenfalls also auch durch die Anwesenheit zusätzlicher Substituenten) substituiert ist.

Sofern keine gezielte Synthese zur Isolierung reiner Isomerer durchgeführt wird, fällt normalerweise ein Produkt als Gemisch zweier optischer Isomerer oder zweier Atropisomerer oder als Gemisch dieser vier möglichen Isomeren an. Die grundsätzlich günstigere fungizide Wirkung der enantiomeren D-Form (im Vergleich zur D, L-Form oder zur L-Form) bleibt jedoch erhalten und wird nicht nennenswert durch die Atropisomerie beeinflußt.

Die nachfolgenden Beispiele dienen zur näheren Erläuterung der Erfindung, ohne dieselbe einzuschränken. Sofern nicht anders vermerkt, ist bei der Nennung eines Wirkstoffs der Formel I, der in optisch aktiven Formen auftreten kann, stets das racemische Gemischt gemeint.

## Beispiel 1

53,6 Teile 2,6-Dimethylpyridin und 74,3 Teile 1,2,3-Thiadiazol-(4)-carbonsäurechlorid wurden parallel über zwei Zuführungen unter Rühren bei 20 bis 25 °C in eine Lösung von 103,7 Teilen N-(1′-Methoxy-carbonyl-ethyl)-2,6-dimethyl-anilin in 350 Teilen Methylenchlorid eingeführt. Nach 1 Stunde Rühren bei 25 °C wurde das Reaktionsgemisch von dem ausgefallenen Hydrochlorid abgesaugt. das Filtrat wurde mit wenig gesättigter Sodalösung und dann dreimal mit 1N Salzsäure gewaschen, über Magnesiumsulfat getrocknet und filtriert. Nach dem Abziehen des Lösungsmittels im Vakuum wurde der verbleibende Rückstand in einer Mischung von Methyl-tert.-Butylether/Petrolether 1 : 3 angerieben und abgesaugt. Nach dem Trocknen schmolzen die farblosen Kristalle des N-(1′-Methoxy-carbonyl-ethyl)-N-(1″,2″,3″-thiadiazol-(4″)-carbonyl)-2,6-dimethyl-anilins bei 86 bis 90 °C.

## Beispiel 2

21 Teile N-(1′-Methoxy-carbonyl-ethyl)-2-methyl-6-ethylanilin und 9,6 Teile Triethylamin wurden über zwei Zufünrungen parallel in eine Lösung von 12,6 Teilen 1,2,3-Thiadiazol-(4)-carbonsäurechlorid in 100 Teilen Essigester unter Rühren bei − 10 bis 0 °C eingeführt. Nach 1 Stunde Rühren bei 20 °C wurde das Reaktionsgemisch dreimal mit 1N Salzsäure und mit Wasser gewaschen. Nach dem Trocknen, Chromatographieren über neutralem Aluminiumoxid und Einengen im Vakuum wurde N-(1′-Methoxy-carbonyl-ethyl)-N-(1″,2″,3″-thiadiazol-(4″)-carbonyl)-2-methyl-6-ethylanilin als leicht gelbliches Öl erhalten, $n_D^{25} = 1,565\,3$.

## Beispiel 3

12,6 Teile 1,2,3-Thiadiazol-(4)-carbonsäurechlorid wurden unter Rühren bei 30 °C innerhalb 10 Minuten zu einer Mischung von 19 Teilen N-(1′-Dimethoxy-propyl-2′)-2,6-dimethylanilin und 10,3 Teilen N,N-Dimethylanilin in 130 Teilen Toluol gegeben. Nach 30 Minuten Rühren bei 30 °C wurde das

Reaktionsgemisch mit wenig gesättigter Sodalösung und dann dreimal mit 1N Salzsäure gewaschen. Nach dem Trocknen und Einengen im Vakuum wurde N-(1'-Dimethoxy-propyl-2')-N-(1",2",3"-thiadiazol-(4")-carbonyl)-2,6-dimethylanilin als leicht gelbliches Öl erhalten, $n_D^{25} = 1{,}571\ 0$.

Beispiel 4

14,1 Teile 1,2,3-Thiadiazol-(4)-carbonsäurechlorid und 8,5 Teile Pyridin wurden parallel über 2 Zuführungen bei 20 bis 35 °C innerhalb 10 Minuten unter Rühren zu einer Mischung von 19,6 Teilen N-(1'-Methoxy-carbonyl-ethyl)-2-methyl-6-chloranilin in 100 Teilen 1,2-Dichlorethan gegeben. Nach 30 Minuten Rühren bei 60 °C wurde das Reaktionsgemisch mit gesättigter Sodalösung und dann dreimal mit 1N Salzsäure gewaschen. Nach dem Trocknen, Chromatographieren über Aluminiumoxid und Einengen im Vakuum wurde N-(1'-Methoxy-carbonyl-ethyl)-N-(1",2",3"-Thiadiazol-(4")-carbonyl)-2-methyl-6-chloranilin in Form farbloser Kristalle erhalten, Fp 59-62 °C.

Analog Beispiel 1 lassen sich folgende Verbindungen der Formel I herstellen :

| Nr. | $R^1$ | $R^2$ | $R^3$ | $R^4$ | Fp °C/$n_D^{25}$ |
|---|---|---|---|---|---|
| 5 | $CH_3$ | H | $CO_2CH_3$ | 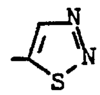 | |
| 6 | $CH_3$ | H | $CH(OCH_3)_2$ | 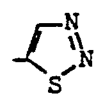 | |
| 7 | $CH_3$ | 3-$CH_3$ | $CO_2CH_3$ | 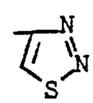 | 1,5610 |

Die erfindungsgemäßen Wirkstoffe weisen eine starke fungitoxische Wirkung auf. Sie schädigen Kulturpflanzen in den zur Bekämpfung von Pilzen und Bakterien notwendigen Konzentrationen nicht. Aus diesen Gründen sind sie für den Gebrauch als Pflanzenschutzmittel zur Bekämpfung von Pilzen geeignet.

Die neuen Wirkstoffe zeigen eine starke fungitoxische Wirsamkeit gegen phytopathogene Pilze. Sie sind beispielsweise geeignet zur Bekämpfung von Erysiphe graminis (echter Mehltau) in Getreide, Erysiphe cichoriacearum (echter Mehltau) an Kürbisgewächsen, Erysiphe polygoni an Bohnen, Podosphaera leucotridia und Phytophthora cactorum an Äpfeln, Phytophtora infestans an Tomaten und Kartoffeln, Phytophthora parasitica an Erdbeeren, Pseudoperonospora cubensis an Gurken, Pseudoperonospora humuli an Hopfen, Peronospora destructor an Zwiebeln, Peronospora tabacina an Tabak, Peronospora sparsa an Rosen, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Uncinula necator an Reben und Sphaerotheca pamosa an Rosen.

Die Aufwandmengen liegen je nach Art des gewünschten Effektes zwischen 0,1 und 5 kg Wirkstoff je ha. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen. Darüber hinaus sind viele der neuen Verbindungen systemisch wirksam, so daß über die Wurzelbehandlung auch ein Schutz oberirdischer Pflanzen möglich ist.

Ferner lassen sich mit den neuen Verbindungen auch Pilze, die Keimlings- und Auflaufkrankheiten hervorrufen, beispielsweise Pythium- und Aphanomyces-Arten an Leguminosen und Baumwolle, bekämpfen. Die Aufwandmengen betragen je 100 kg Saatgut 10 bis 200 g Wirkstoff ; die Anwendung erfolgt in Form von Saatgutbeizmitteln.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemäßen Substanzen können in die üblichen Formulierungen wie Lösungen, Emulsionen, Suspensionsn, Staübe, Pulver, Pasten und Granulate übergeführt werden. Die Anwendungsformen richten sich ganz nach den Verwendungszwecken ; sie sollten in jedem Fall eine feine und gleichmäßige Verteilung der wirksamen Substanzen gewährleisten, da durch eine größere Teilchenfeinheit in den Formulierungen die fungizide Wirksamkeit günstig beeinflußt wird. Die Formulierungen werden in bekannter Weise hergestellt, z. B. durch Verstrecken des Wirkstoffs mit Lösungsmitteln und/oder Trägerstoffen, ggf. unter Verwendung von Emulgiermitteln und Dispergiermitteln, wobei im Falle der Benutzung von Wasser als Verdünnungsmittel auch andere organische Lösungsmittel als

Hilfslösungsmittel verwendet werden können. Als Hilfsstoffe kommen dafür im wesentlichen Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen — Fettalkohol — Ether, Alkylsulfonate und Arylsulfonate) und Dispergiermittel, wie Lignin, Sulfitablaugen und Methylcellulose in Betracht.

Die fungiziden Mittel enthalten 0,1 bis 95 Gew.% Wirkstoff, vorzugsweise 0,5 bis 90 Gew.%.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung des Beispiels 7 mit 10 Gew.-Teilen N-Methyl-α-pyrrolidon und erhält eine Lösung, die zur Anwendung in Form kleinster Tropfen geeignet ist.

II. 20 Gew.-Teile der Verbindung des Beispiels 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.% des Wirkstoffs enthält.

III. 20 Gew.-Teile der Verbindung des Beispiels 4 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

IV. 20 Gew.-Teile der Verbindung des Beispiels 2 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

V. 20 Gew.-Teile der Verbindung des Beispiels 4 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfitablauge und 60 Gew.-Teilen pulverförmigen Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

VI. 3 Gew.-Teile der verbindung des Beispiels 7 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung des Beispiels 4 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

VIII. 40 Gew.-Teile der Verbindung des Beispiels 2 werden mit 10 Teilen Natriumsalz eines Phenolsulfonsäureharnstoff-formaldehyd-Kondensates, 2 Teilen Kieselgel und 48 Teilen Wasser innig vermischt. Man erhält eine stabile wäßrige Dispersion. Durch Verdünnen mit 100 000 Gew.-Teilen Wasser erhält man eine wäßrige Dispersion, die 0,04 Gew.% Wirkstoff enthält.

IX. 20 Teile der Verbindung des Beispiels 4 werden mit 2 Teilen Calciumsalz der Dodecylbenzolsulfonssäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und anderen Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, dit mit den erfindungsgemäßen Verbindungen kombiniert werden können, sind beispielsweise:

Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,
Zinkethylenbisdithiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid ;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;
heterocyclische Strukturen, wie
N-Trichlormethylthio-tetrahydrophthalamid,
N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol

und verschiedene Fungizide, wie
Dodecylguanidinacetat,
3-(3-(3,5-Dimethyl-2-oxycyclohexyl)-2-hydroxyäthyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzœsäure-anilid,
2-Iod-benzœsäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(n-Propyl)-N-(2,4,6-trichlorphenoxyethyl)-N'-imidazolylharnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin.

Beispiel A

Wirksamkeit gegen Phytophthora infestans an Tomaten

Blätter von Tomatenpflanzen der Sorte « Professor Rudloff » werden mit wäßrigen Suspensionen, die 80 % (Gewichtsprozent) des zu prüfenden Wirkstoffs und 20 % Natriumligninsulfonat in der Trockensubstanz enthalten, besprüht. Es werden 0,05- und 0,025 %ige Spritzbrühen (berechnet auf die Trockensubstanz) verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Beispiel B

Wirksamkeit gegen Rebenperonospora

Blätter von Topfreben der Sorte « Müller-Thurgau » werden mit wäßrigen Emulsionen, die 80 % (Gewichtsprozent) des zu prüfenden Wirkstoffs und 20 % Emulgiermittel enthalten, besprüht. Es werden 0,025 %ige Spritzbrühen (bezogen auf die Trockensubstanz) verwendet. Um die Wirkungsdauer der Wirkstoffe beurteilen zu können, werden die Pflanzen nach dem Antrocknen des Spritzbelages zehn Tage im Gewächshaus aufgestellt. Erst dann werden die Blätter mit einer Zoosporenaufschwemmung von Plasmopara viticola (Rebenperonospora) infiziert. Danach kommen die Reben zunächst für 16 Stunden in eine wasserdampfgesättigte Kammer bei 24 °C und anschließend 8 Tage in ein Gewächshaus mit Temperaturen zwischen 20 und 30 °C. Nach dieser Zeit werden die Pflanzen zur Beschleunigung des Sporangienträgerausbruches abermals während 16 Stunden in der feuchten Kammer aufgestellt. Dann erfolgt die Beurteilung des Ausmaßes des Pilzausbruches auf den Blattunterseiten.

Beispiel C

Wirksamkeit gegen Weizenmehltau

Blätter von in Töpfen gewachsenen Weizenkeimlingen der Sorte « Jubilar » werden mit wäßrigen Emulsionen aus 80 % (Gew.%) Wirkstoff und 20 % Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22 °C und 75 bis 80 % relativer Luftfeuchtigkeit aufgestellt. Nach 10 Tagen wird das Ausmaß der Mehltaupilzentwicklung ermittelt.

In den Beispielen A, B und C zeigen insbesondere die Verbindungen der Beispiele 1-3 und 7 wesentlich günstigere Eigenschaften als der bekannte Wirkstoff Furalaxyl (D,L-Methyl-N-(2,6-dimethylphenyl)-N-furoyl(2)-alaninat, DE-OS 25 13 732).

**Ansprüche** (für die Vertragsstaaten : BE, CH, DE, FR, GB, IT, LI, LU, NL, SE)

1. N-Disubstituierte Anilinderivate der Formel I

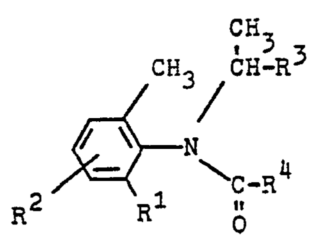

(I)

in der

$R^1$ $C_1$-$C_2$-Alkyl oder Chlor
$R^2$ Wasserstoff oder Methyl
$R^3$ $CH[OCH_3]_2$ oder $COOCH_3$ und
$R^4$ einen 1,2,3-Thiadiazolyl-(4)- oder -(5)-Rest bedeuten.

2. N-(1′-Methoxy-carbonyl-ethyl)-N-(1″,2″,3″-thiadiazol-(4″)-carbonyl)-2,6-dimethylanilin.

3. Verfahren zur Herstellung von N-disubstituierten Anilinderivaten der Formel I gemäß Anspruch 1, dadurch gekennzeichnet, daß man eine Verbindung der Formel II

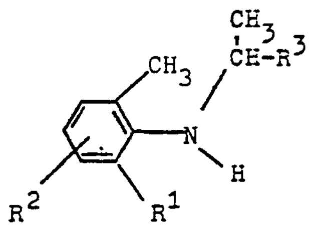

(II)

in der $R^1$, $R^2$ und $R^3$ die in Anspruch 1 genannten Bedeutungen haben, mit einem Carbonsäurederivat der Formel III

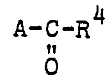

(III)

in der $R^4$ die in Anspruch 1 genannte Bedeutung hat und A für eine nucleophil verdrängbare Abgangsgruppe steht, umsetzt.

4. Mikrobizides Mittel, enthaltend mindestens ein N-disubstituiertes Anilinderivat der Formel I gemäß Anspruch 1.

5. Mikrobizide Mittel, enthaltend mindestens ein N-disubstituiertes Anilinderivat der Formel I gemäß Anspruch 1 und einen festen oder flüssigen Trägerstoff.

6. Verwendung eines N-disubstituierten Anilinderivats der Formel I gemäß Anspruch 1 zur Bekämpfung von Pilzen.

7. Verfahren zur Bekämpfung von Pilzen, dadurch gekennzeichnet, daß man mindestens ein N-disubstituiertes Anilinderivat der Formel I gemäß Anspruch 1 auf diese bzw. auf durch Pilzbefall bedrohte Flächen, Pflanzen oder Saatgüter einwirken läßt.

**Ansprüche** (für der Vertragsstaat AT)

1. Verfahren zur Herstellung von N-disubstituierten Anilinderivaten der Formel I

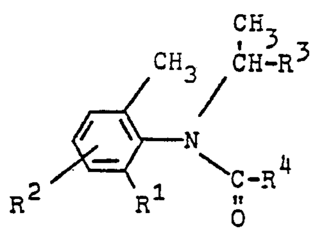

(I)

in der
$R^1$ $C_1$-$C_2$-Alkyl oder Chlor
$R^2$ Wasserstoff oder Methyl
$R^3$ $CH[OCH_3]_2$ oder $COOCH_3$ und
$R^4$ einen 1,2,3-Thiadiazolyl-(4)- oder -(5)-Rest bedeuten,
dadurch gekennzeichnet, daß man eine Verbindung der Formel II

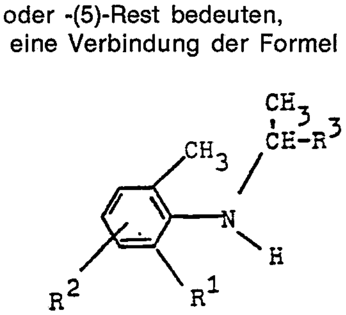

(II)

in der $R^1$, $R^2$ und $R^3$ die in Anspruch 1 genannten Bedeutungen haben, mit einem Carbonsäurederivat der Formel III

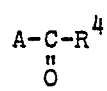

(III)

in der $R^4$ die in Anspruch 1 genannte Bedeutung hat und A für eine nucleophil verdrängbare Abgangsgruppe steht, umsetzt.

2. Verfahren gemäß Anspruch 1, dadurch gekennzeichnet, daß man N-(1'-Methoxy-carbonyl-ethyl)-N-(1",2",3"-thiadiazol-(4")-carbonyl)-2,6-dimethylanilin herstellt.

3. Mikrobizides Mittel, enthaltend mindestens ein N-disubstituiertes Anilinderivat der Formel I gemäß Anspruch 1.

4. Verwendung eines N-disubstituierten Anilinderivats der Formel I gemäß Anspruch 1 zur Bekämpfung von Pilzen.

5. Verfahren zur Bekämpfung von Pilzen, dadurch gekennzeichnet, daß man mindestens ein N-disubstituiertes Anilinderivat der Formel I gemäß Anspruch 1 auf diese bzw. auf durch Pilzbefall bedrohte Flächen, Pflanzen oder Saatgüter einwirken läßt.

**Claims** (for the Contracting States : BE, CH, DE, FR, GB, IT, LI, LU, NL, SE)

1. An N-disubstituted aniline derivative of the formula I

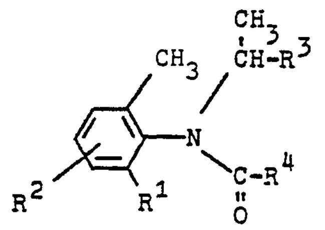

(I)

where

$R^1$ is alkyl of 1 or 2 carbon atoms or chlorine,

$R^2$ is hydrogen or methyl,

$R^3$ is $CH[OCH_3]_2$ or $COOCH_3$, and

$R^4$ is 1,2,3-thiadiazol-4-yl or 1,2,3-thiadiazol-5-yl.

2. N-(1'-methoxy-carbonyl-ethyl)-N-(1",2",3"-thiadiazol-(4")-carbonyl)-2,6-dimethylaniline.

3. A process for manufacturing an N-disubstituted aniline derivative of the formula I as claimed in claim 1, wherein a compound of the formula II

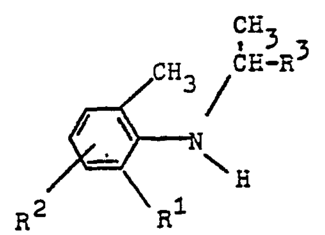

(II)

where $R^1$, $R^2$ and $R^3$ have the meanings given in claim 1, is reacted with a carboxylic acid derivative of the formula III

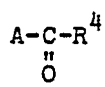

(III)

where $R^4$ has the meanings given in claim 1 and A is a nucleophilically displaceable leaving group.

4. A microbicidal agent containing at least one N-disubstituted aniline derivative of the formula I as claimed in claim 1.

5. A microbicidal agent containing at least one N-disubstituted aniline derivative of the formula I as claimed in claim 1 and a solid or liquid carrier.

6. The use of an N-disubstituted aniline derivative of the formula I as claimed in claim 1 for combating fungi.

7. A process for combating fungi, wherein at least one N-disubstituted aniline derivative of the formula I as claimed in claim 1 is allowed to act on the fungi or areas, plants or seed threatened by fungus attack.

**Claims** (for the Contracting State AT)

1. A process for manufacturing an N-disubstituted aniline derivative of the formula I

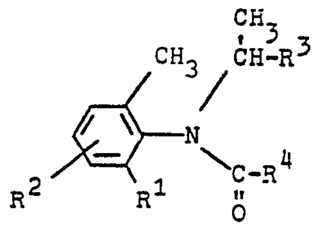

(I)

where

$R^1$ is alkyl of 1 or 2 carbon atoms or chlorine,

$R^2$ is hydrogen or methyl,

$R^3$ is $CH[OCH_3]_2$ or $COOCH_3$, and

$R^4$ is 1,2,3-thiadiazol-4-yl or 1,2,3-thiadiazol-5-yl,

wherein a compound of the formula II

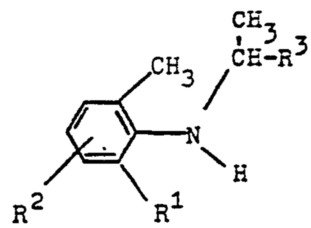

(II)

where $R^1$, $R^2$ and $R^3$ have the meanings given above, is reacted with a carboxylic acid derivative of the formula III

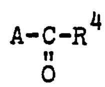

(III)

where $R^4$ has the meanings given above and A is a nucleophilically displaceable leaving group.

2. A process as claimed in claim 1, wherein N-(1′-methoxy-carbonyl-ethyl)-N-(1″,2″,3″-thiadiazol-(4″)-carbonyl)-2,6-dimethylaniline is prepared.

3. A microbicidal agent containing at least one N-disubstituted aniline derivative of the formula I as defined in claim 1.

4. The use of an N-disubstituted aniline derivative of the formula I as defined in claim 1 for combating fungi.

5. A process for combating fungi, wherein at least one N-disubstituted aniline derivative of the formula I as defined in claim 1 is allowed to act on the fungi or areas, plants or seed threatened by fungus attack.

**Revendications** (pour les Etats contractants : BE, CH, DE, FR, GB, IT, LI, LU, NL, SE)

1. Dérivés d'aniline disubstitués sur N, de formule I

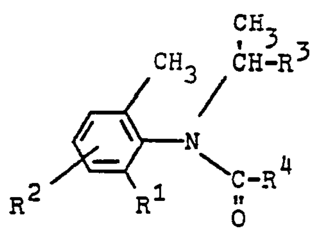

(I)

dans laquelle

$R^1$ représente alkyle en $C_1$-$C_2$ ou chlore

$R^2$, hydrogène ou méthyle

$R^3$, $CH[OCH_3]_2$ ou $COOCH_3$ et

$R^4$, un reste 1,2,3-thiadiazolyle-(4) ou -(5)

2. N-(1′-méthoxy-carbonyl-éthyl)-N-(1″,2″,3″-thiadiazol-(4″)-carbonyl)-2,6-diméthylaniline.

3. Procédé de préparation de dérivés d'aniline disubstitués sur N, de formule I, selon la revendication 1, caractérisé par le fait que l'on fait réagir un composé de formule II

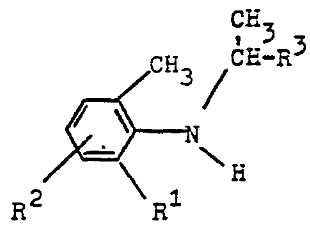

(II)

dans laquelle $R^1$, $R^2$ et $R^3$ ont les significations indiquées dans la revendication 1, avec un dérivé d'acide carboxylique de formule III

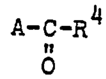

(III)

dans laquelle $R^4$ a la signification donnée dans la revendication 1 et A représente un groupe éliminable, déplaçable, nucléophile.

4. Agent microbicide contenant au moins un dérivé d'aniline disubstitué sur N, de formule I, selon la revendication 1.

5. Agent microbicide contenant au moins un dérivé d'aniline disubstitué sur N, de formule I, selon la revendication 1, et un support solide ou liquide.

6. Utilisation d'un dérivé d'aniline disubstitué sur N, de formule I, selon la revendication 1, pour combattre des champignons.

7. Procédé de lutte contre des champignons, caractérisé par le fait qu'on fait agir au moins un dérivé d'aniline disubstitué sur N, de formule I, selon la revendication 1, sur ces champignons ou sur les surfaces, plantes ou semences menacées par des champignons.

**Revendications** (pour l'Etat contractant AT)

1. Procédé de préparation de dérivés d'aniline disubstitués sur N, de formule I

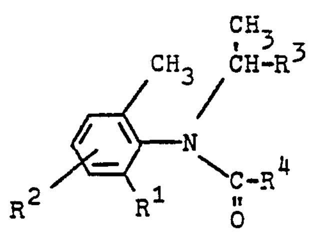

(I)

dans laquelle

$R^1$ représente alkyle en $C_1$-$C_2$ ou chlore

$R^2$, hydrogène ou méthyle

$R^3$, $CH[OCH_3]_2$ ou $COOCH_3$ et

$R^4$, un reste 1,2,3-thiadiazole-(4) ou -(5)

caractérisé par le fait que l'on fait réagir un composé de formule II

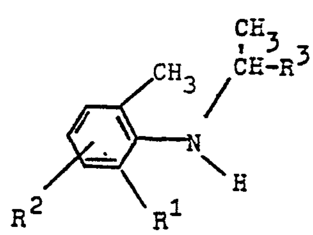

(II)

dans laquelle $R^1$, $R^2$ et $R^3$ ont les significations indiquées dans la revendication 1, avec un dérivé d'acide carboxylique de formule III

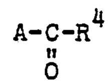

(III)

dans laquelle $R^4$ a la signification donnée dans la revendication 1 et A représente un groupe éliminable, déplaçable, nucléophile.

2. Procédé selon la revendication 1, caractérisé par le fait qu'on prépare du N-(1'-méthoxy-carbonyl-éthyl)-N-(1",2",3"-thiadiazol-(4")-carbonyl)-2,6-diméthylaniline.

3. Agent microbicide contenant au moins un dérivé d'aniline disubstitué sur N, de formule I, selon la revendication 1.

4. Utilisation d'un dérivé d'aniline disubstitué sur N, de formule I, selon la revendication 1, pour combattre des champignons.

5. Procédé de lutte contre des champignons, caractérisé par le fait qu'on fait agir au moins un dérivé d'aniline disubstitué sur N, de formule I, selon la revendication 1, sur ces champignons ou sur les surfaces, plantes ou semences menacées par des champignons.